# EUROPEAN PATENT SPECIFICATION

(11) **EP 4 047 085 B1**
(45) Date of publication and mention of the grant of the patent: **26.02.2025**
(21) Application number: 20897508.6
(22) Date of filing: 24.11.2020
(51) Int. Cl.: C12N 9/10, C12P 13/06

(54) **NOVEL BRANCHED-CHAIN AMINO ACID AMINOTRANSFERASE MUTANT AND LEUCINE PRODUCTION METHOD USING SAME**
NEUARTIGE VERZWEIGTKETTIGE AMINOSÄUREN-AMINOTRANSFERASE-MUTANTE UND VERFAHREN ZUR HERSTELLUNG VON LEUCIN DAMIT
NOUVEAU MUTANT AMINOTRANSFÉRASE D'ACIDE AMINÉ À CHAÎNE RAMIFIÉE ET PROCÉDÉ DE PRODUCTION DE LEUCINE L'UTILISANT

(30) Priority: 06.12.2019 KR 20190161716
(43) Date of publication of application: 24.08.2022
(73) Proprietor: CJ Cheiljedang Corporation, Seoul 04560 (KR)
(72) Inventor: BAE, Hyun-jung, Seoul 04560 (KR); KIM, Ju Eun, Seoul 04560 (KR); BAEK, Min Ji, Seoul 04560 (KR); LEE, Ji Hye, Seoul 04560 (KR)
(74) Representative: Kraus & Lederer PartGmbB
(86) International application number: PCT/KR2020/016675
(87) International publication number: WO 2021/112469

(56) References cited:
- KR-A- 20120 098 235
- KR-B1- 102 143 964
- US-A- 5 120 654
- US-A1- 2003 100 054
- US-A1- 2008 261 278
- US-B2- 7 220 572
- DATABASE REfSeq [online] 1 June 2019 (2019-06-01), "branched-chain amino acid aminotransferase [Corynebacterium caspium]", XP002807963, Database accession no. WP_026209313

## Description

### [Technical Field]

The present disclosure relates to a novel branched-chain amino acid aminotransferase variant and a method for producing leucine using the same.

### [Background Art]

Branched-chain amino acids refer to three types of amino acids (i.e., valine, leucine, and isoleucine), and are known to be metabolized mainly in muscles to be used as an energy source during activity. Since the branched-chain amino acids are known to play an important role in the maintenance and increase of muscles during activity, the amount thereof used is increasing. In particular, leucine is a kind of essential amino acids and is widely used in medicines, foods, feed additives, industrial drugs, *etc.*

Meanwhile, the production of leucine using microorganisms is mainly performed by microorganisms belonging to the genus *Escherichia* or *Corynebacterium* (US 2020-0032305 A1), and is known to biosynthesize 2-ketoisocaproate as a precursor from pyruvate through several steps. However, since enzymes used in the synthesis of leucine are equally used in the biosynthesis of branched-chain amino acids, it is difficult to industrially mass-produce one type of branched-chain amino acid through fermentation.

Database REfSeq WP_026209313 discloses a branched-chain amino acid aminotransferase from Corynebacterium caspium (1 June, 2019).

### [Disclosure]

### [Technical Problem]

The present inventors have made extensive efforts to develop a method for producing leucine with a higher yield compared to the related art, and as a result, they have found a branched-chain amino acid aminotransferase (bcaT) variant that increases leucine productivity, thereby completing the present disclosure.

### [Technical Solution]

An object of the present disclosure is to provide a branched-chain amino acid aminotransferase variant in which a valine (V) amino acid residue at position 156 from an N-terminus of an amino acid sequence of SEQ ID NO: 1 is substituted with another amino acid; a polynucleotide encoding the variant; a vector containing the polynucleotide; and a microorganism containing the vector.

Another object of the present disclosure is to provide a method for producing leucine, comprising culturing the microorganism in a medium.

### [Advantageous Effects]

According to the present disclosure, since the branched-chain amino acid aminotransferase variant increases leucine productivity compared to a wild type, the branched-chain amino acid aminotransferase variant can be widely used for more efficient mass-production of leucine.

### [Best Mode for Carrying Out the Invention]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the present disclosure described herein. Further, these equivalents should be interpreted to fall within the present disclosure.

In a first aspect the present invention relates to a branched-chain amino acid aminotransferase variant, in which a valine (V) amino acid at position 156 from an N-terminus of an amino acid sequence of SEQ ID NO: 1 is substituted with another amino acid, wherein the variant has 80% or more of identity with the amino acid sequence of SEQ ID NO: 1.

In an embodiment, the valine at position 156 is substituted with alanine.

In another embodiment, the variant has 90% or more of identity with the amino acid sequence of SEQ ID NO: 1.

In another embodiment, the variant consists of an amino acid sequence of SEQ ID NO: 3.

In a second aspect the invention relates to a polynucleotide encoding the variant of the first aspect.

In a third aspect, the invention relates to a vector comprising the polynucleotide of the second aspect.

In a fourth aspect, the invention relates to a *Corynebacterium* sp. microorganism comprising at least one of the variant of the first aspect; a polynucleotide encoding the variant; and a vector comprising the polynucleotide.

In one embodiment of the fourth aspect, the *Corynebacterium* sp. microorganism produces leucine.

In another embodiment of the fourth aspect, the *Corynebacterium* sp. microorganism is *Corynebacterium glutamicum.*

In a fifth aspect, the invention relates to a method for producing leucine, comprising culturing the microorganism of the fourth aspect in a medium.

In one embodiment of the fifth aspect, the method further comprises: isolating or recovering leucine from the cultured microorganism or medium.

An aspect of the present disclosure may provide a branched-chain amino acid aminotransferase variant in which a valine (V) amino acid residue at position 156 from an N-terminus of an amino acid sequence of SEQ ID NO: 1 is substituted with another amino acid.

Specifically, the present disclosure may provide a branched-chain amino acid aminotransferase variant comprising one or more substitutions in the amino acid sequence of SEQ ID NO: 1, wherein the amino acid substitution may include a substitution in which the amino acid at position 156 from the N-terminus is substituted with another amino acid other than valine. The "another amino acid" is not limited as long as the amino acid is amino acids other than valine, which is the amino acid at position 156 in the amino acid sequence of SEQ ID NO: 1. Specifically, the variant may be a protein in which the amino acid at position 156 in the amino acid sequence of SEQ ID NO: 1 is substituted with a nonpolar amino acid, and more specifically, the variant may be a branched-chain amino acid aminotransferase variant in which the valine at position 156 in the amino acid sequence of SEQ ID NO: 1 is substituted with alanine, but the variant is not limited thereto.

In the present disclosure, the term "branched-chain amino acid aminotransferase (beaT)" refers to an enzyme involved in the biosynthesis of branched-chain amino acids. In the present disclosure, the branched-chain amino acid aminotransferase may be used interchangeably with "bcaT", "transaminase B" or "ilvE". In addition, the branched-chain amino acid aminotransferase may be encoded by an *ilvEgene,* but is not limited thereto.

In microorganisms, leucine is known to be biosynthesized from pyruvate via acetolactic acid, dihydroxy isovaleric acid, ketoisovaleric acid, 2-isopropylmalic acid, 3-isopropylmalic acid, and ketoisocaproic acid. In addition, such a biosynthetic process is catalyzed and performed by enzymes, such as acetohydroxy acid synthase, acetohydroxyacid isomeroreductase, dihydroxy acid dehydratase, isopropylmalic acid synthase, isopropylmalic acid dehydratase, isopropylmalic acid dehydrogenase, and branched-chain amino acid aminotransferase.

However, since the branched-chain amino acid aminotransferase is also involved in the biosynthesis of valine and isoleucine as well as leucine, there has been a problem when one type of branched-chain amino acids is industrially manufactured through fermentation by manipulating the enzymes.

In the present disclosure, the sequence of the branched-chain amino acid aminotransferase may be obtained from GenBank of a known database (i.e., NCBI), but is not limited thereto, and the branched-chain amino acid aminotransferase may be obtained based on various methods well known in the art. Examples of the methods include gene synthesis technology including codon optimization so as to obtain enzymes with high efficiency from *Corynebacterium* sp. microorganisms, which are commonly used for enzyme expression, or a screening method of useful enzyme resources by bioinformatics methods based on mass genome information of microorganisms, but are not limited thereto.

In the present disclosure, the branched-chain amino acid aminotransferase to be mutated may be branched-chain amino acid aminotransferase derived from a *Corynebacterium* sp. microorganism, and specifically may be a polypeptide/protein including the amino acid sequence of SEQ ID NO: 1, but polypeptides/proteins having the equivalent activity may be included without limitation. An example thereof may include an amino acid sequence of SEQ ID NO: 1 or an amino acid sequence having 80% or more of identity thereto, but is not limited thereto. Specifically, the branched-chain amino acid aminotransferase may include an amino acid sequence having at least 80%, 85%, 90%, 95%, 96%, 97%, 98%, or 99% or more of identity with SEQ ID NO: 1. In addition, if a polypeptide/protein having an amino acid sequence in which part of the sequence is deleted, modified, substituted, or added is also a polypeptide/protein having such homology or identity and exhibiting branched-chain amino acid aminotransferase activity, it is obvious that the polypeptide/protein is included within the range of the polypeptide/protein to be mutated in the present disclosure.

That is, even if the "protein or polypeptide having an amino acid sequence of a specific sequence number" or the "protein or polypeptide consisting of an amino acid sequence of a specific sequence number" is disclosed in the present disclosure, if the protein or polypeptide has the same or corresponding activity as or to a protein or polypeptide consisting of the amino acid sequence of the corresponding sequence number, it is obvious that a protein or polypeptide having an amino acid sequence in which part of the sequence is deleted, modified, substituted, or added may also be used in the present disclosure. For example, even if part of the sequence of the "polypeptide consisting of the amino acid sequence of SEQ ID NO: 1" is deleted, modified, or substituted, or the polypeptide thereof contains the added amino acid sequence, if the polypeptide has the same or corresponding activity as or to "the polypeptide consisting of the amino acid sequence of SEQ ID NO: 1", it is obvious that the polypeptide may belong to the "polypeptide consisting of the amino acid sequence of SEQ ID NO: 1".

In the present disclosure, the term "variant" refers to a protein, in which at least one amino acid in the conservative substitution and/or modification is different from that of the recited sequence, but the functions or properties of the protein are maintained. A variant differs from the sequence identified by several amino acid substitutions, deletions, or additions. Such a variant may generally be identified by modifying one of the polypeptide sequences and by evaluating the properties of the modified polypeptide. That is, the ability of a variant may be increased, unchanged, or reduced compared to that of its native protein. In addition, some variants may include variants in which one or more portions, such as an N-terminal leader sequence or a transmembrane domain, have been removed. Other variants may include variants in which part of the N-terminus and/or C-terminus of a mature protein is removed. In the present disclosure, the term "conservative substitution" may mean substituting one amino acid with another amino acid having similar structural and/or chemical properties. The variant may have, for example, one or more conservative substitutions while still having one or more biological activities. Such amino acid substitutions may generally occur based on similarity in polarity, charge, solubility, hydrophobicity, hydrophilicity, and/or amphipathic nature of residues. Typically, the conservative substitutions may have little or no effect on the activity of the polypeptide.

In addition, the variants may include deletion or addition of amino acids that have a minimal effect on the properties and secondary structure of the polypeptide. For example, a variant or a modified polypeptide may be conjugated with a signal (or leader) sequence at the N-terminus of the protein involved in the transfer of the protein co-translated or post-translated. In addition, the variant may be conjugated with other sequences or linkers to identify, purify, or synthesize the polypeptide.

The term "variant" may also be used interchangeably with "modification", "modified protein", "modified polypeptide", "mutant", "mutein", "divergent", "variant", *etc*., but the term to be used is not limited thereto and any term may be used, as long as it is used in a sense of being mutated. For the purposes of the present disclosure, the variant may be a variant in which branched-chain amino acid aminotransferase activity is changed to enhance the leucine productivity.

That is, in the present disclosure, the term "branched-chain amino acid aminotransferase variant" includes one or more amino acid substitutions in the amino acid sequence of the polypeptide having the branched-chain amino acid aminotransferase activity, and the term "branched-chain amino acid aminotransferase variant" may be used interchangeably with the term "polypeptide having the branched-chain amino acid aminotransferase activity". In addition, the term "branched-chain amino acid aminotransferase variant" may be used interchangeably with "modified branched-chain amino acid aminotransferase protein", "modified bcaT", "modified bcaT protein", "bcaT variant", "modified transaminase B protein", "modified transaminase B", "transaminase B variant", "modified ilvE protein", "modified ilvE", "ilvE variant", *etc.,* but is not limited thereto as long as it is used in a sense of being mutated as described above.

It is obvious that the branched-chain amino acid aminotransferase variant, in which the valine (V) amino acid residue at position 156 from the N-terminus of the amino acid sequence of SEQ ID NO: 1 is substituted with another amino acid, may include a protein variant in which an amino acid corresponding to position 156 from the N-terminus of the amino acid sequence of SEQ ID NO: 1 is substituted with another amino acid in the amino acid sequence of the polypeptide having the branched-chain amino acid aminotransferase activity. For example, the variant may be a variant comprising a sequence in which the amino acid corresponding to position 156 from the N-terminus of the amino acid sequence of SEQ ID NO: 1 is substituted with another amino acid in a sequence having at least 80%, 85%, 90%, 95%, 97%, or 99% or more of identity with the amino acid sequence of SEQ ID NO: 1.

The "another amino acid" may be an amino acid other than the amino acid corresponding to the amino acid at position 156 of the amino acid sequence of SEQ ID NO: 1. Specifically, the variant may be a variant in which the amino acid corresponding to the amino acid at position 156 of the amino acid sequence of SEQ ID NO: 1 is substituted with a non-polar amino acid having structural and/or chemical properties similar to alanine. More specifically, the variant may be a variant in which an amino acid corresponding to the amino acid at position 156 of the amino acid sequence of SEQ ID NO: 1 is substituted with alanine, but is not limited thereto.

In the present disclosure, the term "corresponding position" refers to an amino acid residue at a position recited in a protein or polypeptide, or an amino acid residue similar to, identical to, or homologous to a residue recited in the protein or polypeptide. As used herein, the term "corresponding region" generally refers to a similar or corresponding position in a related protein or a reference protein.

In the present disclosure, specific numbering may be used at an amino acid residue position in the protein used in the present disclosure. For example, by aligning the polypeptide sequence of the protein of the present disclosure with a target protein to be compared, it is possible to renumber a position corresponding to the amino acid residue position of the protein of the present disclosure.

The branched-chain amino acid aminotransferase variant of the present disclosure may have activity for increasing the leucine productivity in microorganisms compared to the wild-type or non-variant branched-chain am ino acid am inotransferase protein.

The branched-chain amino acid aminotransferase variant may be one in which the valine (V) amino acid residue at position 156 from the N-terminus of the amino acid sequence of SEQ ID NO: 1 is substituted with another amino acid, more specifically substituted with alanine. In addition, the branched-chain amino acid aminotransferase variant may have 80%, 85%, 90%, 95%, 97%, or 99% or more of identity with the amino acid sequence of SEQ ID NO: 1, more specifically 90% or more of identity with the amino acid sequence of SEQ ID NO: 1.

In one embodiment, the variant may consist of the amino acid sequence of SEQ ID NO: 3, but is not limited thereto. Additionally, in the branched-chain amino acid aminotransferase variant, the amino acid corresponding to position 156 from the N-terminus of the amino acid sequence of SEQ ID NO: 3 is fixed (that is, the amino acid at the position corresponding to the amino acid at position 156 of the amino acid sequence of SEQ ID NO: 3 is alanine like the amino acid at position 156 of the amino acid sequence of SEQ ID NO: 3) and may include an amino acid sequence having 70% or more, specifically 80%, 85%, 88%, 89%, 90%, 91%, 92%, 93%, 94%, 95 %, 96%, 97%, 98%, or 99% or more of identity with SEQ ID NO: 3, but is not limited thereto. In addition, if the variant is an amino acid sequence having such homology or identity and exhibiting efficacy corresponding to the protein, in addition to the amino acid sequence at position 156, it is obvious that a protein having an amino acid sequence in which part of the sequence is deleted, modified, substituted or added is also included within the scope of the present disclosure.

In the present disclosure, the term "homology" or "identity" refers to a degree of relevance between two given amino acid sequences or nucleotide sequences and it may be expressed as a percentage. These terms "homology" and "identity" may often be used interchangeably.

Sequence homology or identity of conserved polynucleotides or polypeptides can be determined by standard alignment algorithm, and default gap penalties established by a program being used may be used together. Actually, homologous or identical sequences may generally hybridize with each other along the entire sequence or at least about 50%, 60%, 70%, 80%, or 90% or more of the entire length under moderate or highly stringent conditions. In hybridization, polynucleotides including a degenerate codon instead of a codon are also considered.

Whether any two polynucleotide- or polypeptide sequences have a homology, similarity, or identity can be determined using computer algorithms known in the art, e.g., "FASTA" program using default parameters introduced by Pearson et al. (1988) [Proc. Natl. Acad. Sci. USA 85: 2444]. Alternatively, Needleman-Wunsch algorithm (1970, J. Mol. Biol. 48: 443-453) performed in a Needleman program of The European Molecular Biology Open Software Suite of EMBOSS package (Rice et al., 2000, Trends Genet. 16: 276-277) (version 5.0.0 or a later version) may be used to determine the same (including GCG program package (Devereux, J., et al., Nucleic Acids Research 12: 387 (1984)), BLASTP, BLASTN, FASTA (Atschul, [S.] [F.,] [ET AL., J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, the homology, similarity, or identity can be determined using BLAST from the National Center for Biotechnology Information database or ClustalW

The homology, similarity, or identity between polynucleotides or polypeptides may be determined, for example, by comparing the given sequence information using a GAP computer program, such as a program introduced by Needleman et al. (J Mol Biol. 48: 443 (1970)), as disclosed by Smith and Waterman (Adv. Appl. Math (1981) 2: 482). In brief, the GAP program defines a homology, similarity, or identity as the number of similar aligned symbols (*i.e.,* nucleotides or amino acids) divided by the total number of the symbols in a shorter one of the two sequences. The default parameters for the GAP program may include: (1) a binary comparison matrix (including a value 1 for identity and a value 0 for non-identity) and the weighted comparison matrix of Gribskov, et al., (Nucl. Adds Res. 14: 6745 (1986)) as described by Schwartz and Dayhoff, eds. (Atlas Of Protein Sequence And Structure, National Biomedical Research Foundation, pp. 353-358 (1979) or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps. Accordingly, the term "homology" or "identity" used in the present disclosure may represent relevance between sequences.

Another aspect of the present disclosure may provide a polynucleotide encoding the branched-chain amino acid aminotransferase variant.

In addition, still another aspect of the present disclosure may provide a vector comprising the polynucleotide.

As used herein, the term "polynucleotide" refers to a DNA or RNA strand having more than a certain length as a nucleotide polymer, which is a long chain of nucleotide monomers linked by a covalent bond, and more specifically refers to a polynucleotide fragment encoding the branched-chain amino acid aminotransferase variant described above.

The polynucleotide encoding the branched-chain amino acid aminotransferase variant of the present disclosure may be included without limitations as long as the polynucleotide is a polynucleotide sequence encoding the branched-chain amino acid aminotransferase variant of the present disclosure. The polynucleotide encoding the branched-chain amino acid aminotransferase variant may be included without limitations as long as the polynucleotide is a sequence encoding a variant in which the amino acid at position 156 of the amino acid sequence of SEQ ID NO: 1 is substituted with another amino acid. Specifically, the polynucleotide may be a polynucleotide sequence encoding a variant in which the amino acid at position 156 of the amino acid sequence of SEQ ID NO: 1 is substituted with alanine. For example, the polynucleotide encoding the branched-chain amino acid aminotransferase variant of the present disclosure may be a polynucleotide sequence encoding the amino acid sequence of SEQ ID NO: 3, but is not limited thereto. More specifically, the polynucleotide may consist of a polynucleotide sequence of SEQ ID NO: 4, but is not limited thereto.

Considering codon degeneracy or the codons preferred in a bioorganism where the protein is to be expressed, various modifications may be performed in the coding region of the polynucleotide encoding the protein variant of the present disclosure within the scope not altering the amino acid sequence of the protein. Therefore, it will be apparent that a polynucleotide that may be translated into the polynucleotide consisting of the amino acid sequence of SEQ ID NO: 3 or the polypeptide having homology or identity thereto by codon degeneracy may also be included.

Additionally, any sequence which encodes a branched-chain amino acid aminotransferase variant, in which the 156^{th} amino acid of SEQ ID NO: 1 is substituted with a different amino acid, by hybridizing with any probe that can be prepared from known gene sequences (e.g., complementary sequences to all or part of the above base sequence) under stringent conditions, can be included without limitation.

The term "stringent conditions" refers to conditions which enables specific hybridization between polynucleotides. Such conditions are specifically described in references (e.g., J Sambrook et al., Molecular Cloning, A Laboratory Manual, 2nd Edition, Cold Spring Harbor Laboratory press, Cold Spring Harbor, New York, 1989). For example, the references may recite conditions of hybridizing between genes having high homology or identity, i.e., genes having homology or identity of 80% or more, 85% or more, specifically 90 % or more, more specifically 95 % or more, and much more specifically 97% or more, and particularly specifically 99% or more and not hybridizing between genes having homology or identity lower therethan, or washing conditions of general southern hybridization, in which washing is performed once, particularly 2 to 3 times at a salt concentration and a temperature corresponding to 60°C, 1 X SSC, 0.1% SDS, particularly 60°C, 0.1 X SSC, 0.1% SDS, and more particularly 68°C, 0.1 X SSC, 0.1% SDS.

Hybridization requires that two nucleic acids include complementary sequences, although mismatches between bases are possible depending on the stringency of the hybridization. The term "complementary" is used to describe the relationship between mutually-hybridizable nucleotide bases. For example, with respect to DNA, adenine is complementary to thymine, and cytosine is complementary to guanine. Accordingly, the present disclosure may also include isolated nucleic acid fragments complementary to the entire sequence as well as substantially similar nucleic acid sequences.

Specifically, polynucleotides having homology or identity may use a hybridization condition including hybridization at a Tm value of 55°C and may be detected using the aforementioned condition. In addition, the Tm value may be 60°C, 63°C, or 65°C, but is not limited thereto, and the Tm value may be properly adjusted by those skilled in the art according to a purpose thereof.

A proper stringent degree for hybridizing the polynucleotides depends on the length and the complementary degree of the polynucleotide and the variables are well-known in the art (see Sambrook *et al.,* supra, 9.50-9.51, 11.7-11.8).

In the present disclosure, the term "vector" may refer to a DNA construct including a nucleotide sequence of a polynucleotide encoding a target modified protein, which is operably linked to a suitable control sequence so that the target modified protein can be expressed in a suitable host. The control sequence may include a promoter capable of initiating transcription, any operator sequence for controlling the transcription, a sequence encoding an appropriate mRNA ribosome-binding site, and a sequence for controlling the termination of transcription and translation. The vector, after being transformed into a suitable host cell, may be replicated or function irrespective of the host genome, or may be integrated into the genome itself.

The vector used in the present disclosure is not particularly limited, and may be any vector known in the art. Examples of the vector to be generally used may include plasmids, cosmids, viruses, and bacteriophages which are in a native or recombinant state. For example, as the phage vector or cosmid vector, pWE15, M13, MBL3, MBL4, IXII, ASHII, APII, t10, t11, Charon4A, Charon21A, and the like may be used; and as the plasmid vector, pBR-based, pUC-based, pBluescriptll-based, pGEM-based, pTZ-based, pCL-based, and pET-based plasmids may be used. Specifically, as the plasmid vector, pDZ, pACYC177, pACYC184, pCL, pECCG117, pUC19, pBR322, pMW118, pCC1BAC vectors, and the like may be used, but are not limited thereto.

For example, the polynucleotide encoding a target modified protein in the chromosome may be replaced with a mutated polynucleotide through a vector for intracellular chromosomal insertion. The insertion of a polynucleotide into the chromosome may be performed using any method known in the art (e.g., homologous recombination), but the method is not limited thereto. The polynucleotide may further include a selection marker for confirming its successful insertion into the chromosome. A selection marker is used for selection of cells transformed with the vector, i.e., to confirm whether the target nucleic acid molecule has been inserted, and markers which confer selectable phenotypes (e.g., drug resistance, auxotrophy, resistance to cytotoxic agents, expression of surface modified proteins, *etc*.) may be used. Under the circumstances where selective agents are treated, only the cells capable of expressing the selection markers can survive or express other phenotypic traits, thereby enabling easy selection of the transformed cells.

Still another aspect of the present disclosure may provide a microorganism containing at least one of the branched-chain amino acid aminotransferase variant; a polynucleotide encoding the variant; and a vector containing the polynucleotide.

Specifically, the microorganism may be a microorganism produced by transformation with the vector containing the polynucleotide encoding the variant, but is not limited thereto.

In the present disclosure, the term "transformation" means that the protein encoding the polynucleotide may be expressed in the host cell by introducing a vector including a polynucleotide encoding a target protein into the host cell. As long as the transformed polynucleotide may be expressed in the host cell, all transformed polynucleotides may be included regardless of whether the transformed polynucleotide is inserted and positioned in the chromosome of the host cell or positioned out of the chromosome. In addition, the polynucleotide includes DNA and RNA encoding the target protein. The polynucleotide may be introduced in any form as long as the polynucleotide may be introduced into the host cell and expressed. For example, the polynucleotide may be introduced into the host cell in the form of an expression cassette, which is a genomic structure including all elements required to be self-expressed. The expression cassette may generally include a promoter which is operably linked to the polynucleotide, a transcription termination signal, a ribosome-binding site, and a translation termination signal. The expression cassette may be a self-replicable expression vector form. Further, the polynucleotide may also be introduced into the host cell in its own form to be operably linked to a sequence required for expression in the host cell, but is not limited thereto.

In addition, the term "operably linked" above may mean that the gene sequence is functionally linked to a promoter sequence, which initiates and mediates the transcription of the polynucleotide encoding the branched-chain amino acid aminotransferase of the present disclosure.

The microorganism of the present disclosure may be a microorganism which has leucine productivity and which comprises at least one of the branched-chain amino acid aminotransferase variant; a polynucleotide encoding the variant; and a vector comprising the polynucleotide, but is not limited thereto. The microorganism may be a microorganism having natural leucine productivity, or a microorganism having leucine productivity given to a parent strain which does not have leucine productivity, but is not limited thereto.

For the purposes of the present disclosure, the microorganism may be any microorganism capable of producing leucine by expressing the branched-chain amino acid aminotransferase variant.

As used herein, the term "protein allowed to be expressed/to be expressed/expressed" may refer to a state in which the target protein is introduced into the microorganism or is modified to be expressed in the microorganism. When the target protein is a protein present in the microorganism, it may mean a state in which its activity is enhanced, as compared with the endogenous activity or activity before modification. With respect to the objects of the present disclosure, the "target protein" may be the branched-chain amino acid aminotransferase variant described above.

Specifically, "introduction of the protein" may mean that a microorganism exhibits activity of a particular protein that was not originally possessed, or exhibits enhanced activity, as compared with the endogenous activity of the corresponding protein or the activity before modification. For example, a polynucleotide encoding a particular protein may be introduced into a chromosome in a microorganism, or a vector comprising a polynucleotide encoding a particular protein may be introduced into a microorganism to exhibit its activity.

In addition, "enhancement of the activity" may mean that the activity is improved compared to the endogenous activity or pre-modification activity of the specific protein included in the microorganism. The "endogenous activity" may mean the activity of the specific protein which has been originally possessed by the parent strain before the transformation when the microorganism is transformed due to genetic variation caused by natural or artificial factors. In one embodiment, the enhancement of the protein activity may be achieved by enhancing the expression of the gene encoding the protein, but is not limited thereto.

Specifically, in the present disclosure, the activity enhancement of the protein variant may be performed by at least one method selected from the group consisting of a method of increasing the intracellular copy number of the gene encoding the protein variant, a method of introducing a mutation into an expression regulatory sequence of the gene encoding the protein variant, a method of replacing the gene expression regulatory sequence encoding the protein variant with a sequence having strong activity, a method of replacing a gene encoding a native protein having branched-chain amino acid aminotransferase activity in a chromosome with a gene encoding the protein variant, a method of additionally introducing a mutation into a gene encoding the variant so that the activity of the protein variant is enhanced, and a method of introducing a protein variant into a microorganism, but is not limited thereto.

Next, the modification of the expression regulatory sequence for increasing expression of a polynucleotide may be, but is not particularly limited to, performed by inducing a modification in the polynucleotide sequence via deletion, insertion, non-conservative or conservative substitution, or a combination thereof so as to further enhance the activity of the expression regulatory sequence, or by replacing the polynucleotide sequence with a nucleotide sequence with a stronger activity. The expression regulatory sequence may include, but is not particularly limited to, a promoter, an operator sequence, a sequence encoding a ribosome-binding domain, and a sequence regulating termination of transcription and translation, *etc.*

A strong promoter instead of an original promoter may be linked to the upper portion of the polynucleotide expression unit, but is not limited thereto. Examples of the known strong promoter include cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but are not limited thereto.

Further, the modification of the polynucleotide sequence on the chromosome is not particularly limited, but may be performed by inducing a mutation on the expression regulatory sequence by deletion, insertion, non-conservative or conservative substitution, or a combination thereof of a nucleic acid sequence to further enhance the activity of the polynucleotide sequence, or may be performed by replacement with a polynucleotide sequence improved to have stronger activity.

In the introduction and enhancement of the protein activity, the activity or concentration of the corresponding protein may be generally increased to at least 1 %, 10%, 25%, 50%, 75%, 100%, 150%, 200%, 300%, 400%, or 500%, at most 1,000% or 2,000% based on the activity or concentration of the protein in a wild-type or non-modified microorganism strain, but is not limited thereto.

The microorganism may include all microorganisms capable of expressing a variant which is transformed with a vector containing a protein variant or a polynucleotide encoding the protein variant or a polynucleotide encoding the variant. Specific examples of the microorganism may include microbial strains such as *Escherichia* sp., *Serratia* sp., *Erwinia* sp., *Enterobacteria* sp., *Salmonella* sp., *Streptomyces* sp., *Pseudomonas* sp., *Brevibacterium* sp., or *Corynebacterium* sp., specifically *Corynebacterium* sp. microorganisms, and more specifically *Corynebacterium glutamicum,* but are not limited thereto.

The microorganism of the present disclosure may be a microorganism producing leucine.

In the present disclosure, the term "microorganism producing leucine" may be microorganisms having natural leucine productivity or microorganisms in which the leucine productivity is given to a parent strain which does not have leucine productivity. In addition, the microorganism producing leucine may include a microorganism genetically modified through at least one of the branched-chain amino acid aminotransferase variant, a polynucleotide encoding the variant, and a vector containing the polynucleotide; a microorganism modified to express the branched-chain amino acid aminotransferase variant or a polynucleotide encoding the variant; and a recombinant microorganism expressing the branched-chain amino acid aminotransferase variant or a polynucleotide encoding the variant, but is not limited thereto.

The microorganism producing leucine is a microorganism, which includes a polynucleotide encoding a protein variant, or a microorganism, which is capable of expressing a variant which is transformed with a vector containing a polynucleotide encoding a variant, and for the purposes of the present disclosure, the microorganism may include all microorganisms capable of producing leucine by containing an ilvE variant. Specific examples of the microorganism may include microbial strains such as *Escherichia* sp., *Serratia* sp., *Erwinia* sp., *Enterobacteria* sp., *Salmonella* sp., *Streptomyces* sp., *Pseudomonas* sp., *Brevibacterium* sp., or *Corynebacterium* sp., specifically *Corynebacterium* sp. microorganisms, and more specifically *Corynebacterium glutamicum,* but are not limited thereto.

In the present disclosure, the term *"Corynebacterium* sp. microorganism producing leucine" may mean *Corynebacterium* sp. microorganisms having leucine productivity through a wild type or mutation. It has been known that the *Corynebacterium* sp. microorganism may produce leucine to some extent, but the leucine productivity is remarkably low and it is difficult to produce the *Corynebacterium* sp. microorganism industrially. Therefore, in the present disclosure, the *Corynebacterium* sp. microorganism having leucine productivity may mean *Corynebacterium* sp. microorganisms that have improved leucine productivity by a native microorganism itself or inserting a gene related to an external leucine production mechanism or enhancing, attenuating, or inactivating the activity of an intrinsic gene. For the purposes of the present disclosure, the microorganism producing leucine may be a microorganism characterized in that the leucine productivity is increased by including the branched-chain amino acid aminotransferase variant of the present disclosure. In one embodiment, the microorganism may have increased leucine productivity compared to a microorganism that does not contain a natural wild-type strain, a parent strain, or the variant of the present disclosure.

In the present disclosure, the term "pre-modified strain" or "pre-modified microorganism" does not exclude strains containing mutations that may occur naturally in microorganisms, and may mean either a wild-type strain or a natural-type strain itself, or a strain before the trait is changed by genetic mutation caused by natural or artificial factors. The "pre-modified strain" or "pre-modified microorganism" may be used interchangeably with "non-mutated strain", "non-modified strain", "non-mutated microorganism", "non-modified microorganism" or "reference microorganism".

In one embodiment, the *Corynebacterium* sp. microorganisms may be *Corynebacterium* sp. microorganisms which have enhanced expression of a gene involved in a leucine biosynthetic pathway or attenuated/inactivated expression of a gene involved in a leucine decomposition pathway, and specifically may be *Corynebacterium* sp. microorganisms producing leucine, which have additionally enhanced isopropylmalate synthase activity, but are not limited thereto. The enhancement of the protein activity is as described above.

The term "attenuation/inactivation of the protein activity" in the present disclosure may mean that the expression of an enzyme or protein is not expressed at all compared to a natural wild type strain, a parent strain, or a strain in which the corresponding protein is non-modified or the activity thereof is absent or decreased even if expressed. In particular, the decrease is a concept including a case where the activity of the protein is decreased compared to the activity of the protein originally possessed by the microorganism due to mutation of the gene encoding the protein, modification of the expression regulatory sequence, or deletion of part or all of the gene, a case where the overall protein activity degree in the cells is lower than that of the native strain or the pre-modified strain due to inhibition of expression or translation of the gene encoding the same, and a combination thereof. In the present disclosure, the inactivation/attenuation may be achieved by applying various methods well-known in the art. Examples of the method may include 1) a method of deleting all or part of the gene encoding the protein; 2) modification of the expression regulatory sequence to reduce the expression of the gene encoding the protein; 3) modification of the gene sequence encoding the protein so that the activity of the protein is eliminated or attenuated; 4) introduction of an antisense oligonucleotide (e.g., antisense RNA) that complementarily binds to a transcript of the gene encoding the protein; 5) a method of disabling attachment of ribosome by forming a secondary structure by adding a sequence complementary to a Shine-Dalgamo sequence to the front of the Shine-Dalgarno sequence of the gene encoding the protein; 6) a method (reverse transcription engineering, RTE) of adding a promoter transcribed in an opposite direction to a 3' terminus of an open reading frame (ORF) of a polynucleotide sequence of the gene encoding the protein, *etc.* The method may also be achieved by combinations thereof, but is not particularly limited thereto. inactivation/attenuation methods known in the art may be appropriately selected and applied.

Still another aspect of the present disclosure may provide a method for producing leucine, comprising culturing the microorganism.

In the method, the culturing of the microorganism is not particularly limited, but may be performed by a known batch culture method, a continuous culture method, a fed-batch culture method, or the like. In particular, the culture condition is not particularly limited, but an aerobic condition may be maintained by adjusting an appropriate pH (*e.g.,* pH 5 to 9, specifically pH 6 to 8, and most specifically pH 6.8) using a basic compound (*e.g.,* sodium hydroxide, potassium hydroxide, or ammonia) or an acidic compound (*e.g.,* phosphoric acid or sulfuric acid) and by introducing oxygen or an oxygen-containing gas mixture into the culture medium. The culture temperature may be maintained at 20°C to 45°C, specifically 25°C to 40°C, and the microorganism may be cultured for about 10 hours to 160 hours, but is not limited thereto. Leucine produced by the culture above may be secreted into the medium or may remain in the cells.

In addition, in the culture medium used, as a carbon source, sugar and carbohydrates (*e.g.,* glucose, sucrose, lactose, fructose, maltose, molasse, starch, and cellulose), oils and fats (*e.g.,* soybean oil, sunflower seed oil, peanut oil, and coconut oil), fatty acids (*e.g.,* palmitic acid, stearic acid, and linoleic acid), alcohols (*e.g.,* glycerol and ethanol), organic acids (*e.g.,* acetic acid), *etc.* may be used individually or in combination, but the carbon source is not limited thereto. As a nitrogen source, nitrogen-containing organic compounds (*e.g.,* peptone, yeast extract, meat juice, malt extract, corn steep liquor, soybean meal, and urea), inorganic compounds (*e.g.,* ammonium sulfate, ammonium chloride, ammonium phosphate, ammonium carbonate, and ammonium nitrate), or the like may be used individually or in combination, but the nitrogen source is not limited thereto. As a phosphorus source, potassium dihydrogen phosphate, dipotassium hydrogen phosphate, sodium-containing salts corresponding thereto, *etc.* may be used individually or in combination, but the phosphorus source is not limited thereto. In addition, the medium may contain essential growth-promoting substances such as other metal salts (*e.g.,* magnesium sulfate or iron sulfate), amino acids, and vitamins.

The production method may further include a step of recovering leucine from the cultured microorganism or culture medium.

The method of isolating or recovering leucine may collect a desired amino acid from the culture medium using a suitable method known in the art according to the culture method. For example, centrifugation, filtration, anion exchange chromatography, crystallization, HPLC, *etc.* may be used, and it is possible to collect desired leucine from the medium or microorganism by using a proper method known in the art.

Still another aspect of the present disclosure may provide a method for increasing leucine productivity, comprising modifying a microorganism so as to express a branched-chain amino acid aminotransferase variant in which a valine (V) amino acid residue at position 156 from an N-terminus of an amino acid sequence of SEQ ID NO: 1 is substituted with another amino acid.

Still another aspect of the present disclosure may provide use for increasing leucine production of the protein variant.

The protein variant is as described above.

### [Mode for Carrying out the Invention]

Hereinafter, the present disclosure will be described in more detail with reference to Examples and Experimental Examples. However, these Examples and Experimental Examples are only illustrative of the present disclosure, and the scope of the present disclosure is not limited to these Examples and Experimental Examples.

### Example 1: Discovery of ilvE variant

### 1-1. Preparation of vector containing ilvE

In order to prepare an ilvE variant library having the activity of branched-chain amino acid aminotransferase, first, a recombinant vector including part of ilvE was first prepared. An amino acid sequence and a nucleotide sequence of wild-type ilvE were the same as SEQ ID NO: 1 and SEQ ID NO: 2, respectively. PCR was performed using the chromosomal DNA of *Corynebacterium glutamicum* ATCC13032 as a template along with the primers of SEQ ID NO: 5 and SEQ ID NO: 6, and the amplified product was cloned into *E. coli* vector pCR2.1 using a TOPO cloning kit (Invitrogen) to obtain pCR-ilvE.

### 1-2. Preparation of ilvE variant library

An ilvE variant library was prepared based on the vector prepared in Example 1-1. The library was prepared using an error-prone PCR kit (clontech Diversify^{®} PCR Random Mutagenesis Kit). Under conditions where the variant may occur, PCR was performed using the primers of SEQ ID NO: 5 and SEQ ID NO: 6. Specifically, after pre-heating at 94°C for 30 seconds under a condition that 0 to 3 variants occurred per 1,000 bp, the process of 30 seconds at 94°C and 1 minute and 30 seconds at 68°C was repeated 25 times. The PCR product obtained at this time was treated with Dpnl after the process of 95°C for 50 seconds, 60°C for 50 seconds, and 68°C for 12 minutes was repeated 25 times by using a megaprimer (500 ng to 125 ng), transformed into *E*. *coli* DH5α, and smeared on an LB solid medium containing kanamycin (25 mg/L). After the 20 transformed colonies were screened, the plasmid was obtained and the polynucleotide sequence was analyzed, and as a result, it was confirmed that variants were introduced into different positions at a frequency of 2 mutations/kb. About 20,000 transformed *E. coli* colonies were taken to extract the plasmid, which was named pTOPO-ilvE-library.

### Example 2: Library evaluation and variant screening in leucine-producing strain

In general, even if a wild-type *Corynebacterium* sp. strain produces leucine, only a very small amount of leucine is produced. Thus, a leucine-producing strain derived from ATCC13032 was prepared, and the variants were screened by introducing the library vector prepared in Example 1-2.

### 2-1. Preparation of leucine-producing strain CA13-8100

In order to produce a leucine-producing strain derived from wild-type *Corynebacterium glutamicum* ATCC13032, an isopropylmalate synthase (hereinafter, referred to as "IPMS") variant was introduced to prepare a CA13-8100 strain.

Specifically, the variant included a mutation in which G, which was a nucleotide at position 1673 of a *leuA* gene encoding isopropylmaleate synthase, was substituted with A, and arginine, which was an amino acid at position 558 of the IPMS protein, was substituted with histidine, and a mutation in which nucleotides at positions 1682 and 1683, GC are substituted with AT and glycine, an amino acid at position 561, was substituted with aspartic acid (US 2020-0032305 A1).

The pDC-leuA (R558H, G561D) vector containing the leuA mutation was transformed into wild-type ATCC13032, and a strain into which the vector was inserted on the chromosome by recombination of a homologous sequence was screened in a medium containing 25 mg/L of kanamycin.

The screened primary strain was again subjected to a secondary cross-over, and a strain into which the mutation of the *leuA* gene was introduced was selected. Whether the mutation in the final transformed strain was introduced was confirmed by performing PCR using the primers of SEQ ID NO: 7 and SEQ ID NO: 8, followed by analyzing a base sequence. As a result, it was confirmed that the mutation was introduced into the final transformed strain.

An ATCC13032_leuA_(R558H, G561D) strain transformed with the pDC-leuA(R558H, G561D) vector was named CA13-8100.

### 2-2. Library evaluation and screening of variant

The pTOPO-ilvE-library prepared in Example 1-2 was transformed into the leucine-producing strain CA13-8100 prepared in Example 2-1 by electroporation, and then smeared on a nutrient medium containing 25 mg/L of kanamycin to obtain 10,000 colonies of the strain into which the mutant gene was inserted, and each colony was named from CA13-8100/pTOPO_ilvE(mt)1 to CA13-8100/pTOPO_ilvE(mt)10000.

Fermentation titer was evaluated for each colony in the following method in order to identify colonies with increased leucine production among the 10,000 colonies obtained.
- **Nutrient medium**: Glucose 10 g, Meat juice 5 g, Polypeptone 10 g, Sodium chloride 2.5 g, Yeast extract 5 g, Agar 20 g, Urea 2 g (based on 1 liter of distilled water)
- **Production medium:** Glucose 50 g, Ammonium sulfate 20 g, Corn steep solids 20 g, Dipotassium phosphate 1 g, Magnesium sulfate heptahydrate 0.5 g, Biotin 100 µg, Thiamine-HCl 1 mg, Calcium carbonate 15 g (based on 1 liter of distilled water), pH 7.0

Each colony was inoculated into 250 mL of a comer-baffle flask containing 25 µg/mL of kanamycin in 25 mL of an autoclaved production medium with a platinum loop, and then shake-cultured at 200 rpm at 30°C for 60 hours. After completion of the culture, the amount of leucine production was measured by a method using high-performance liquid chromatography (HPLC, SHIMAZDU LC20A), and one strain having the most improved leucine productivity compared to the CA13-8100 strain was screened. The concentration of leucine produced in the screened strain is shown in Table 1 below.

**[Table 1]**

| Strain name | Leucine (g/L) |
|---|---|
| CA13-8100 | 3.4 |
| CA13-8100/pTOPO_ilvE(mt)3012 | 3.97 |

### 2-3. Confirmation of mutations in screened variant strain

In order to confirm the genetic mutation of the strain screened in Example 2-2, PCR was performed on the CA13-8100/pTOPO_ilvE(mt)3012 strain using the primers of SEQ ID NO: 9 and SEQ ID NO: 10, the sequencing was performed, and the *ilvE* gene was compared with *ilvE* of a wild-type ATCC1 3032, and it was confirmed that the strain contained a mutation in the *ilvE* gene.

Specifically, it was confirmed that in the CA13-8100/pTOPO_ilvE(mt)3012 strain, T (i.e., the nucleotide at position 503 of the *ilvE* gene) was substituted with C (SEQ ID NO: 4). This is a mutation in which valine at position 156 in the amino acid sequence of ilvE is substituted with alanine (SEQ ID NO: 3). Therefore, in the following Examples, it was confirmed whether the mutation affected the amount of leucine production of the Corynebacterium sp. microorganism.

### Example 3: Confirmation of leucine productivity of ilvE screening variant

### 3-1. Preparation of insertion vector containing ilvE variant

In order to introduce the variants screened in Example 2 into the strain, an insertion vector was prepared. The vector for introducing the ilvE (V156A) variant was prepared using a site directed mutagenesis method. PCR was performed using the chromosomal DNA of *Corynebacterium glutamicum* ATCC13032 as a template along with the primer pair of SEQ ID NOs: 11 and 12 and SEQ ID NOs: 13 and 14. After denaturation at 94°C for 5 minutes, the PCR was performed by 30 cycles of denaturation at 94°C for 30 seconds, annealing at 55°C for 30 seconds, and polymerization at 72°C for 1 minute 30 seconds, and then polymerization was performed at 72°C for 5 minutes. As a result, the obtained gene fragments were cloned by fusing a homologous sequence of 15 bases at the terminal between the DNA fragments using an In-Fusion enzyme with a linear pDC vector cleaved by restriction enzymes Pstl and Xbal to prepare a pDC-ilvE (V156A) vector substituting an amino acid at position 156, valine (Val), with alanine (Ala).

### 3-2. Introduction of ilvE variant in CA13-8100 strain and ATCC13032 and confirmation of leucine productivity

A leucine-producing strain, CA13-8100, was transformed with the pDC-ilvE (V156A) vector prepared in Example 3-1, and the strain in which the vector was inserted into the chromosome by recombination of the homologous sequence was screened in a medium containing 25 mg of kanamycin. The screened primary strain was again subjected to a secondary cross-over, and strains into which the mutation of the target gene was introduced were selected. Whether the ilvE gene mutation in the final transformed strain was introduced was confirmed by performing PCR using the primers of SEQ ID NO: 9 and SEQ ID NO: 10 and then analyzing a base sequence. As a result, it was confirmed that the ilvE mutation was introduced into the final transformed strain. The prepared CA13-8100_ilvE_V156A was named as CA13-8107.

In addition, the wild-type *Corynebacterium glutamicum* ATCC13032 was transformed with the pDC-ilvE (V156A) vector by the same method, and the prepared ATCC13032_ilvE_V156A strain was named as CA13-8106.

The leucine productivity of the prepared CA13-8106 and CA13-8107 strains was evaluated. Flask culture was performed in the same manner as in Example 2-2, and after completion of the culture, the amount of leucine production was measured by a method using HPLC, and the culture results are shown in Table 2 below.

**[Table 2]**

| Strain name | Leucine (g/L) |
|---|---|
| ATCC13032 | 0.14 |
| ATCC13032_ilvE_V156A : CA13-8106 | 0.19 |
| ATCC13032_leuA_(R558H, G561D): CA13-8100 | 3.4 |
| CA13-8100_ilvE_V156A: CA13-8107 | 4.1 |

As shown in Table 2, it was confirmed that the leucine-producing strain, *Corynebacterium glutamicum* CA13-8100, had significantly improved leucine productivity compared to its parent strain, *Corynebacterium glutamicum* ATCC13032. In addition, it was confirmed that the CA13-8107 strain introduced with the ilvE V156A mutation in the CA13-8100 strain improved the leucine productivity by 120% compared to the parent strain, CA13-8100. In addition, it was confirmed that the CA13-8106 strain introduced with the mutation based on the wild-type ATCC13032 also increased leucine production by 135% compared to the wild-type.

Through the results above, it can be confirmed that the amino acid at position 156 of the amino acid sequence of ilvE (i.e., branched-chain amino acid aminotransferase) is an important position for ilvE enzyme activity.

The CA13-8107 strain was deposited on November 15, 2019, with the Korean Culture Center of Microorganisms (KCCM), an international depository under the Budapest Treaty, and was given with the deposit number KCCM12630P.

### 3-3. Introduction of ilvE variant into Corynebacterium glutamicum KCCM11661P and KCCM11662P strains and confirmation of leucine productivity

The recombinant vector pDC-ilvE(V156A) prepared in Example 3-1 was transformed into *Corynebacterium glutamicum* KCCM11661P (US 10351859 B2) and *Corynebacterium glutamicum* KCCM11662P (US10351859 B2), which are leucine-producing strains, by homologous recombination on the chromosome. The two strains are variants having leucine productivity by treating wild-type *Corynebacterium glutamicum* ATCC 14067 and *Corynebacterium glutamicum* ATCC 13869 with N-methyl-N'-nitro-N-nitrosoguanidine (NTG). It was confirmed that the mutant was introduced into the *ilvE* gene by performing PCR for the final transformed strains using the primers of SEQ ID NO: 9 and SEQ ID NO: 10, followed by analyzing a base sequence. The recombinant strains were named *Corynebacterium glutamicum* KCCM11661P_ilvE_V156A and *Corynebacterium glutamicum* KCCM11662P_ilvE_V156A, respectively. In order to confirm the leucine productivity of the strain, flask culture was performed in the same manner as in Example 2-2, and after the culture was completed, the amount of leucine production was measured by a method using HPLC, and the measured concentrations of leucine are shown in Table 3 below.

**[Table 3]**

| Strain name | Leucine (g/L) |
|---|---|
| KCCM11661P | 2.7 |
| KCCM11661P_ilvE_V156A | 3.3 |
| KCCM11662P | 3.0 |
| KCCM11662P_ilvE_V156A | 3.6 |

Through the results above, it can be confirmed that the amino acid at position 156 of the amino acid sequence of ilvE (*i.e*., branched-chain amino acid aminotransferase) is an important position for ilvE enzyme activity. Particularly, it was confirmed that when the amino acid at the position above was substituted with another amino acid, the leucine productivity was improved.

## Claims

1. A branched-chain amino acid aminotransferase variant, in which a valine (V) amino acid at position 156 from an N-terminus of an amino acid sequence of SEQ ID NO: 1 is substituted with another amino acid, wherein the variant has 80% or more of identity with the amino acid sequence of SEQ ID NO: 1.

2. The branched-chain amino acid aminotransferase variant of claim 1,
wherein the valine at position 156 is substituted with alanine.

3. The branched-chain amino acid aminotransferase variant of claim 1,
wherein the variant has 90% or more of identity with the amino acid sequence of SEQ ID NO: 1.

4. The branched-chain amino acid aminotransferase variant of claim 1,
wherein the variant consists of an amino acid sequence of SEQ ID NO: 3.

5. A polynucleotide encoding the variant of any one of claims 1 to 4.

6. A vector comprising the polynucleotide of claim 5.

7. A *Corynebacterium* sp. microorganism comprising at least one of the variant of any one of claims 1 to 4; a polynucleotide encoding the variant; and a vector comprising the polynucleotide.

8. The *Corynebacterium* sp. microorganism of claim 7, wherein the *Corynebacterium* sp. microorganism produces leucine.

9. The *Corynebacterium* sp. microorganism of claim 7, wherein the *Corynebacterium* sp. microorganism is *Corynebacterium glutamicum.*

10. A method for producing leucine, comprising culturing the microorganism of claim 7 in a medium.

11. The method for producing leucine of claim 10, further comprising: isolating or recovering leucine from the cultured microorganism or medium.

## Patentansprüche

1. Verzweigtkettige Aminosäureaminotransferase-Variante, in der eine Valin (V)-Aminosäure an Position 156 aus einem N-Terminus einer Aminosäuresequenz von SEQ ID NO:1 durch eine andere Aminosäure ersetzt ist, wobei die Variante eine 80%ige oder höhere Identität mit der Aminosäuresequenz von SEQ ID NO:1 aufweist.

2. Verzweigtkettige Aminosäureaminotransferase-Variante nach Anspruch 1, wobei das Valin an Position 156 durch Alanin ersetzt ist.

3. Verzweigtkettige Aminosäureaminotransferase-Variante nach Anspruch 1, wobei die Variante eine 90%ige oder höhere Identität mit der Aminosäuresequenz von SEQ ID NO:1 aufweist.

4. Verzweigtkettige Aminosäureaminotransferase-Variante nach Anspruch 1, wobei die Variante aus einer Aminosäuresequenz von SEQ ID NO:3 besteht.

5. Polynukleotid, das die Variante nach einem der Ansprüche 1 bis 4 kodiert.

6. Vektor, umfassend das Polynukleotid nach Anspruch 5.

7. *Corynebacterium* sp.-Mikroorganismus, umfassend mindestens eine der Varianten nach einem der Ansprüche 1 bis 4, ein die Variante kodierendes Polynukleotid und einen Vektor, der das Polynukleotid umfasst.

8. *Corynebacterium* sp.-Mikroorganismus nach Anspruch 7, wobei der *Corynebacterium* sp.-Mikroorganismus Leucin produziert.

9. *Corynebacterium* sp.-Mikroorganismus nach Anspruch 7, wobei der *Corynebacterium* sp.-Mikroorganismus *Corynebacterium glutamicum* ist.

10. Verfahren zum Produzieren von Leucin, umfassend das Kultivieren des Mikroorganismus nach Anspruch 7 in einem Medium.

11. Verfahren zum Produzieren von Leucin nach Anspruch 10, ferner umfassend: das Isolieren oder Gewinnen von Leucin aus dem kultivierten Mikroorganismus oder Medium.

## Revendications

1. Variante de l'aminotransférase d'acide aminé à chaîne ramifiée, dans laquelle un acide aminé valine (V) en position 156 de l'extrémité N-terminale d'une séquence d'acides aminés de SEQ ID NO : 1 est substitué par un autre acide aminé, dans lequel le variant a 80% ou plus d'identité avec la séquence d'acides aminés de SEQ ID NO : 1.

2. Variante de l'aminotransférase d'acide aminé à chaîne ramifiée de la revendication 1, dans laquelle la valine en position 156 est remplacée par l'alanine.

3. Variante de l'aminotransférase d'acide aminé à chaîne ramifiée de la revendication 1, dans laquelle la variante présente une identité de 90 % ou plus avec la séquence d'acides aminés de SEQ ID NO : 1.

4. Variante de l'aminotransférase d'acide aminé à chaîne ramifiée de la revendication 1, dans laquelle la variante consiste en une séquence d'acides aminés de SEQ ID NO : 3.

5. Polynucléotide codant pour le variant de l'une quelconque des revendications 1 à 4.

6. Vecteur comprenant le polynucléotide de la revendication 5.

7. Micro-organisme *Corynebacterium* sp. comprenant au moins un des variants de l'une quelconque des revendications 1 à 4, un polynucléotide codant pour le variant et un vecteur comprenant le polynucléotide.

8. Micro-organisme *Corynebacterium* sp. de la revendication 7, dans lequel le micro-organisme *Corynebacterium* sp. produit de la leucine.

9. Micro-organisme *Corynebacterium* sp. de la revendication 7, dans lequel le micro-organisme *Corynebacterium* sp. est *Corynebacterium glutamicum.*

10. Procédé de production de leucine, comprenant la culture du micro-organisme de la revendication 7 dans un milieu.

11. Procédé de production de leucine selon la revendication 10, comprenant en outre : l'isolement ou la récupération de la leucine à partir du micro-organisme cultivé ou du milieu.
